# EUROPEAN PATENT APPLICATION

(11) **EP 4 566 668 A2**
(43) Date of publication of application: **11.06.2025**
(21) Application number: 25156529.7
(22) Date of filing: 19.12.2018
(51) Int. Cl.: A61P 17/02

(54) **NEUROTOXINS FOR USE IN MINIMIZING SCARRING**

(30) Priority: 18.12.2017 US 201762599972 P
(62) Divisional of application: 18847260.9
(71) Applicant: Bonti, Inc., Madison, NJ 07940 (US)
(72) Inventor: Abushakra, Sawsan, Madison, New Jersey, 07940 (US); Ahmad, Wajdie, Madison, New Jersey, 07940 (US); Hasan, Fauad, Madison, New Jersey, 07940 (US); Jarpe, Michael, Madison, New Jersey, 07940 (US)
(74) Representative: Hoffmann Eitle

(57) **Abstract**

The present invention provides a fast-acting neurotoxin for use in a method of minimizing scarring, the method comprising administering a therapeutically effective amount of the fast-acting neurotoxin to a patient in the proximity of a wound.

## Description

### CROSS REFERENCE TO RELATED APPLICATIONS

This application claims the benefit of priority to U.S. Provisional Patent Application No. 62/599,972, filed on December 18, 2017, which is incorporated herein by reference in its entirety.

### TECHNICAL FIELD

The present disclosure relates to the use of neurotoxins to minimize scarring.

### BACKGROUND

Surgical and other invasive procedures or injuries can result in scarring. While mechanically immobilizing a tissue or joint can be effective in minimizing scarring, there remains a need for methods that improve clinical outcome related to scar formation associated with wounds.

### SUMMARY

Disclosed herein are compositions and methods for use in minimizing scarring. For example, disclosed embodiments comprise use of a "fast-acting" botulinum toxin to reduce muscle tension in the proximity of a wound, thus preventing or reducing scarring.

In a first aspect, a method for minimizing scarring is provided. The method comprises administering a therapeutically effective amount of a fast-acting neurotoxin to a patient in the proximity of a wound.

In another aspect, a method for prophylaxis of scar formation in patients undergoing a surgical procedure is provided. The method comprises administering a therapeutically effective amount of a fast-acting neurotoxin to a patient in the proximity of a wound created in the surgical procedure.

In another aspect, a method for rapidly reducing the dynamic tension around a wound is provided. The method comprises administering a therapeutically effective amount of a fast-acting and/or short-duration neurotoxin to a patient in the proximity of a wound.

In another aspect, a method for prophylaxis of scar formation in a patient who has recently undergone a surgical procedure and before a scar of the wound is visible is provided. The method comprises administering a therapeutically effective amount of a fast-acting neurotoxin to a patient in the proximity of a wound created in the surgical procedure.

In another aspect, a method for treating a scar in a patient that already has a scar is provided. The method comprises administering a therapeutically effective amount of a fast-acting and/or short-duration neurotoxin to a patient in the proximity of a wound.

In one embodiment, the fast- acting neurotoxin comprises botulinum neurotoxin serotype E.

In one embodiment, the therapeutically effective amount comprises an amount of between about 10⁻³ U/kg and about 35 U/kg. In another embodiment, the therapeutically effective amount comprises an amount of between about 1 U/kg and about 25 U/kg. In still another embodiment, the therapeutically effective amount comprises an amount of between about 5 U/kg and about 15 U/kg.

In yet another embodiment, the therapeutically effective amount comprises an amount of between about 0.2 nanograms and about 2 nanograms.

In another embodiment, muscle activity in the proximity of a skin incision or laceration is reduced, thus reducing or preventing scar formation.

In another embodiment, the botulinum toxin is a fast-recovery toxin.

In yet another embodiment, the "fast-acting" botulinum toxin is also a fast-recovery toxin.

In still another embodiment, the method further comprises administration of a fast-acting botulinum neurotoxin in combination with, for example, a slower-acting neurotoxin. In one embodiment, the slower-acting neurotoxin is botulinum toxin subtype A (BoNT/A).

In other embodiments, the method further comprises administration of a fast-recovery botulinum neurotoxin in combination with, for example, a slower-recovery neurotoxin.

In another embodiment, the method comprises additional surgical procedures. For example, disclosed embodiments comprise administration of a fast-acting botulinum neurotoxin in combination with, for example, a dermal filler injection, an eye lift, rhinoplasty, breast augmentation, an open fracture, treatment of a laceration, or the like.

The fast onset of muscle relaxing effect with BoNT/E offers improved wound healing and reduced scar formation in subjects, such as those undergoing facial laceration repair. The shorter duration of muscle relaxation (2-4 weeks) offered by BoNT/E is also desirable compared to 3-4 months with BoNT/A products.

In another aspect, a method to reduce scar tissue formation to improve scar appearance is provided. The method comprises administering in the proximity of a wound a formulation comprising a botulinum toxin type E in an amount effective to decrease dynamic tension on the wound, and/or to paralyze the muscles in the proximity of the wound. In one embodiment, administering BoNT/E reduces scar tissue formation of the wound relative to a similar wound left untreated or treated with botulinum toxin type A (BoNT/A).

In one embodiment, the wound is created for removal of basal cell carcinoma or squamous cell carcinoma in a subject. In another embodiment, the wound is created during Mohs surgery.

In yet another embodiment, the wound is on the face. In other embodiments, the wound is around the eyes, on the forehead, on the nose, on the scalp, or around an ear.

In still another embodiment, the wound is a single wound on the forehead.

In other embodiments, the formulation or composition comprising BoNT/E is administered intramuscularly.

In one embodiment, the formulation is administered after Mohs surgery. In another embodiment, the formulation is administered prior to Mohs surgery.

In another embodiment, the formulation is administered within about 10 minutes to about 1 hour after Mohs surgery.

In other embodiments, scar appearance is evaluated by a clinician using VAS (visual analog scale). In another embodiment, scar appearance is evaluated by the subject using a Scar Cosmesis Assessment and Rating Scale (SCARS). In still another embodiment, scar appearance is evaluated by the subject using a Patient and Observer Scar Assessment Scale.

In another aspect, a method to improve scar appearance is provided. The method comprises administering in the proximity of a wound a formulation comprising a botulinum toxin type E in an amount effective to reduce redness, reduce stiffness and/or reduce discoloration of the scar, where the reduction in redness, stiffness and/or discoloration are observed in the first 48 hours, 72 hours, 1 week or 1 month of wound healing. In one embodiment, the reductions are assessed by the patient and/or by an observer, such as a clinician, and are relative to a similar wound left untreated or treated with botulinum toxin type A (BoNT/A).

In another aspect, a method to reduce scar tissue formation on a wound is provided. The method comprises subdermally administering in the proximity of a wound a formulation comprising a botulinum toxin type E, whereby said administering reduces scar tissue formation of the wound relative to a similar wound left untreated or treated with botulinum toxin type A.

In one embodiment, the wound is perpendicular to a relaxed skin tension line.

In an embodiment, the botulinum toxin type E administered in an amount effective to decrease dynamic tension to the wound. In another embodiment, the wound is a surgical incision.

In yet another aspect, a method for improving appearance of a scar that forms over a wound is provided. The method comprises administering a therapeutically effective amount of botulinum toxin subtype E (BoNT/E) in the proximity of a wound.

In one embodiment, the wound is a surgical wound. In another embodiment, the BoNT/E is administered before a surgical procedure. In yet another embodiment, the BoNT/E is administered after a surgical procedure.

In one embodiment, the surgical wound overlaps with an existing scar on the subject.

In one embodiment, the therapeutically effective amount comprises an amount that reduces redness of the scar, relative to a scar that forms over a similar wound in a patient not treated with BoNT/E.

In still another embodiment, the therapeutically effective amount comprises an amount that reduces stiffness of the scar, relative to a scar that forms over a similar wound in a patient not treated with BoNT/E.

In yet another embodiment, an amount of botulinum toxin subtype A in the proximity of the wound is also administered before, during or after wound creation.

### BRIEF DESCRIPTION OF THE DRAWINGS

FIG. 1 shows a depiction of the primary structure of a botulinum neurotoxin (BoNT).
FIGS. 2A-2D are schematic and ribbon representations of BoNT/E (FIGS. 2A, 2C) and BoNT/B (FIGS. 2B, 2D). The catalytic, translocation, and binding domains are labeled CD, TD, and BD, respectively.
FIGS. 3A-3B are illustrations of the human body, anterior view (FIG. 3A) and posterior view (FIG. 3B), identifying the muscles.
FIG. 4 is an illustration of the human body with the nerves identified.
FIG. 5 shows forehead injection sites for a study described in Example 1.
FIG. 6 shows the forehead injection sites for a study described in Example 4.
FIG. 7A is a bar graph showing the average change of investigator rated Visual Analog Scale (VAS) on day 2-8, day 2-30 and day 2-90 in human patients treated with BoNT/E intramuscularly in the frontalis muscle during Mohs surgery (bars with dashed fill) and in human patients not treated with botulinum toxin as part of a Mohs surgery (placebo, open bars).
FIG. 7B is a bar graph showing the average change of patient-reported color component of scar assessment using the Patient and Observer Scar Assessment Scale (POSAS) on day 2-8, day 2-30 and day 2-90 in human patients treated with BoNT/E intramuscularly in the frontalis muscle during Mohs surgery (bars with dashed fill) and in human patients not treated with botulinum toxin as part of a Mohs surgery (placebo, open bars).
FIG. 7C is a bar graph showing the average change of patient-reported stiffness component of scar assessment using the Patient and Observer Scar Assessment Scale (POSAS) on day 2-8, day 2-30 and day 2-90 in human patients treated with BoNT/E intramuscularly in the frontalis muscle during Mohs surgery (bars with dashed fill) and in human patients not treated with botulinum toxin as part of a Mohs surgery (placebo, open bars).

### DETAILED DESCRIPTION

Embodiments disclosed herein can reduce local muscular activity and thereby reduce the development of scars, for example scars resulting from surgery. In some embodiments, the surgery can comprise cosmetic surgery, for example rhinoplasty, an eye lift, a "tummy" tuck, or the like. In other embodiments, the surgery can comprise other types of medical procedures, for example appendix removal, organ transplant, cesarean delivery, and the like. In some embodiments, the methods comprise administering disclosed compositions in proximity to a wound.

Cosmetic results of wound repairs are dependent on many factors including the quality of the closure, infections, and suture quality. A key factor is the tension on the wound edges, which tends to increase inflammation, leading to fibrosis and erythema. Tension is composed of static tension (the result of wound location, size, closure techniques, etc.) and dynamic tension. This dynamic tension is caused by the action of the muscles underlying the wound. Specifically, wounds that are repaired parallel to relaxed skin tension lines (RSTL) have less dynamic tension and heal with a better cosmetic result. Scars that are perpendicular to these RSTL have increased tension, which results in increased inflammation, increased deposition of collagen and glycosaminoglycans, increased fibroblastic response, and poor wound healing and increased scarring. Decreasing dynamic tension by denervating the underlying muscle with botulinum toxin offers an intriguing way to potentially lessen scarring.

In some embodiments, the surgery can comprise surgery to treat cancer, for example skin cancer, for example, Mohs surgery. Mohs micrographic surgery is considered the most effective technique for treating many basal cell carcinomas (BCCs) and squamous cell carcinomas (SCCs), including those in cosmetically and functionally important areas around the eyes, nose, lips, ears, scalp, fingers, toes or genitals.

BCCs are abnormal, uncontrolled growths or lesions that arise in the skin's basal cells, which line the deepest layer of the epidermis (the outermost layer of the skin). BCCs often look like open sores, red patches, pink growths, shiny bumps, or scars and are usually caused by a combination of cumulative and intense, occasional sun exposure. More than 4 million cases of basal cell carcinoma are diagnosed in the U.S. each year. In fact, BCC is the most frequently occurring form of all cancers. More than one out of every three new cancers are a skin cancer, and the vast majority are BCCs.

SCCs are a common form of skin cancer that develops in the squamous cells that make up the middle and outer layer of the skin. They are usually not life- threatening, though they can be aggressive in some cases. Untreated, squamous cell carcinoma of the skin can grow large or spread to other parts of the body, causing serious complications. Most SCCs result from prolonged exposure to ultraviolet (UV) radiation, either from sunlight or from tanning beds or lamps. Avoiding UV light helps reduce the risk of squamous cell carcinoma of the skin and other forms of skin cancer. Squamous cells are found in many places in the body and squamous cell carcinoma can occur anywhere squamous cells are found. Squamous cell carcinoma of the skin refers to cancer that forms in the squamous cells found in the skin.

Mohs is also recommended for BCCs or SCCs that are large, aggressive or growing rapidly, that have indistinct edges, or have recurred after previous treatment. Some surgeons are also successfully using Mohs surgery on certain cases of melanoma. The procedure is done in stages, including lab work, while the patient waits. This allows the removal of all cancerous cells for the highest cure rate while sparing healthy tissue. The procedure has up to 99% cure rate for a skin cancer that has not been treated before, and up to 94% cure rate for a skin cancer that has recurred after previous treatment.

The procedure has been used in the removal of melanoma-in-situ (cure rate 77% to 98% depending on surgeon), and certain types of melanoma (cure rate 52%). Another study of melanoma-in-situ revealed Mohs cure rate of 95% for frozen section Mohs, and 98 to 99% for fixed tissue Mohs method.

The Mohs procedure is a pathology sectioning method that allows for the complete examination of the surgical margin. It is different from the standard "bread loafing" technique of sectioning, where random samples of the surgical margin are examined. Mohs surgery is performed in four steps:
a. Surgical removal of tissue (Surgical Oncology)
b. Mapping the piece of tissue, freezing and cutting the tissue between 5 and 10 micrometers using a cryostat, and staining with hematoxylin and eosin (H&E) or other stains (Including Toluidine Blue)
c. Interpretation of microscope slides (Pathology)
d. Possible reconstruction of the surgical defect (Reconstructive Surgery)

The Mohs procedure is usually performed in a physician's office under local anesthetic. A small scalpel is utilized to cut around the visible tumor. A very small surgical margin is utilized, usually with 1 to 1.5 mm of "free margin" or uninvolved skin. The amount of free margin removed is much less than the usual 4 to 6 mm required for the standard excision of skin cancers. After each surgical removal of tissue, the specimen is processed, cut on the cryostat and placed on slides, stained with H&E and then read by the Mohs surgeon/pathologist who examines the sections for cancerous cells. If cancer is found, its location is marked on the map (drawing of the tissue) and the surgeon removes the indicated cancerous tissue from the patient. This procedure is repeated until no further cancer is found. The vast majority of cases are then reconstructed by the Mohs surgeon.

Embodiments disclosed herein can reduce local muscular activity and thereby reduce the development of scars, for example scars resulting from trauma. For example, following a traumatic injury, disclosed embodiments can comprise administering disclosed compositions in proximity to trauma, for example a laceration or amputation.

In some embodiments, the surgery can comprise cosmetic surgery, for example rhinoplasty, an eye lift, a "tummy" tuck, or the like. In other embodiments, the surgery can comprise other types of medical procedures, for example appendix removal, organ transplant, caesarian delivery, and the like. In some embodiments, methods comprise administering disclosed compositions in proximity to a wound.

Administration sites useful for practicing disclosed embodiments can comprise any area where muscle activity is to be reduced. For example, when employed in combination with facial cosmetic surgery procedures, disclosed embodiments can include administration to the glabellar complex, including the corrugator supercilli and the procerus; the obicularis oculi; the superolateral fibers of the obicularis oculi; the frontalis; the nasalis; the levator labii superioris aleque nasi; the obicularis oris; the masseter; the depressor anguli oris; and the platysma.

When employed in combination with other surgical procedures, disclosed embodiments can include administration to, for example, muscles of the arm, leg, torso, and the like.

Disclosed embodiments can comprise methods for preparing a surgical site prior to the procedure, in order to reduce muscle tension in the proximity of an incision.

In some embodiments, compositions disclosed herein can comprise fast-acting botulinum toxins, for example, botulinum type E.

In some embodiments, compositions disclosed herein can comprise fast-recovery botulinum toxins, for example, botulinum type E.

In some embodiments, compositions disclosed herein can comprise fast acting, fast-recovery botulinum toxins, for example, botulinum type E.

### Definitions

"Administration," or "to administer" means the step of giving (i.e. administering) a pharmaceutical composition or active ingredient to a subject. The pharmaceutical compositions disclosed herein can be administered via a number of appropriate routes. For example, intramuscular, intradermal, subcutaneous administration, intrathecal administration, intraperitoneal administration, topical (transdermal), instillation, and implantation (for example, of a slow-release device such as polymeric implant or miniosmotic pump) can all be appropriate routes of administration.

"Alleviating" means a reduction in the occurrence of a pain, of a headache, or of any symptom or cause of a condition or disorder. Thus, alleviating includes some reduction, significant reduction, near total reduction, and total reduction.

The term "amino acid" means a naturally occurring or synthetic amino acid, as well as amino acid analogs, stereoisomers, and amino acid mimetics that function similarly to the naturally occurring amino acids. Included by this definition are natural amino acids such as: (1) histidine (His; H) (2) isoleucine (Ile; I) (3) leucine (Leu; L) (4) Lysine (Lys; K) (5) methionine (Met; M) (6) phenylalanine (Phe; F) (7) threonine (Thr; T) (8) tryptophan (Trp; W) (9) valine (Val; V) (10) arginine (Arg; R) (11) cysteine (Cys; C) (12) glutamine (Gln; Q) (13) glycine (Gly; G) (14) proline (Pro; P) (15) serine (Ser; S) (16) tyrosine (Tyr; Y) (17) alanine (Ala; A) (18) asparagine (Asn; N) (19) aspartic acid (Asp; D) (20) glutamic acid (Glu; E) (21) selenocysteine (Sec; U); including unnatural amino acids: (a) citrulline (Cit); (b) cystine; (c) gama-amino butyric acid (GABA); (d) ornithine (Orn); (f) theanine; (g) homocysteine (Hey); (h) thyroxine (Thx); and amino acid derivatives such as betaine; carnitine; carnosine creatine; hydroxy tryptophan; hydroxyproline (Hyp); N-acetyl cysteine; S-Adenosyl methionine (SAM-e); taurine; tyramine.

"Animal protein free" means the absence of blood derived, blood pooled and other animal derived products or compounds. "Animal" means a mammal (such as a human), bird, reptile, fish, insect, spider or other animal species. "Animal" excludes microorganisms, such as bacteria. Thus, an animal protein free pharmaceutical composition can include a botulinum neurotoxin. For example, an "animal protein free" pharmaceutical composition means a pharmaceutical composition which is either substantially free or essentially free or entirely free of a serum derived albumin, gelatin and other animal derived proteins, such as immunoglobulins. An example of an animal protein free pharmaceutical composition is a pharmaceutical composition which comprises or which consists of a botulinum toxin (as the active ingredient) and a suitable polysaccharide as a stabilizer or excipient.

"Botulinum toxin" or "botulinum neurotoxin" means a neurotoxin produced by Clostridium botulinum, as well as a botulinum toxin (or the light chain or the heavy chain thereof) made recombinantly by a non-Clostridial species. The phrase "botulinum toxin", as used herein, encompasses the botulinum toxin serotypes A, B, C, D, E, F, G, H and X, and their subtypes, mosaic toxins, such as BoNT/DC and BoNT/CD, and any other types of subtypes thereof, or any re-engineered proteins, analogs, derivatives, homologs, parts, sub-parts, variants or versions, in each case, of any of the foregoing. "Botulinum toxin", as used herein, also encompasses a "modified botulinum toxin". Further "botulinum toxin" as used herein also encompasses a botulinum toxin complex, (for example, the 300, 600 and 900kDa complexes), as well as the neurotoxic component of the botulinum toxin (150 kDa) that is unassociated with the complex proteins. "Purified botulinum toxin" means a pure botulinum toxin or a botulinum toxin complex that is isolated, or substantially isolated, from other proteins and impurities which can accompany the botulinum toxin as it is obtained from a culture or fermentation process. Thus, a purified botulinum toxin can have at least 95%, and more preferably at least 99% of the non-botulinum toxin proteins and impurities removed.

"Clostridial neurotoxin" refers to any toxin produced by a Clostridial toxin strain that can execute the overall cellular mechanism whereby a Clostridial toxin intoxicates a cell and encompasses the binding of a Clostridial toxin to a low or high affinity Clostridial toxin receptor, the internalization of the toxin/receptor complex, the translocation of the Clostridial toxin light chain into the cytoplasm and the enzymatic modification of a Clostridial toxin substrate. Non-limiting examples of Clostridial toxins include Botulinum toxins, such as a BoNT/A, a BoNT/B, a BoNT/C₁, a BoNT/D, a BoNT/CD, a BoNT/DC, a BoNT/E, a BoNT/F, a BoNT/G, a BoNT/H (also known as type FA or HA), a BoNT/X, a Tetanus toxin (TeNT), a Baratii toxin (BaNT), and a Butyricum toxin (BuNT). The BoNT/C₂ cytotoxin and BoNT/C₃ cytotoxin, not being neurotoxins, are excluded from the term "Clostridial toxin." The term Clostridial toxin also includes the approximately 150-kDa Clostridial toxin alone (i.e. without the NAPs). A Clostridial toxin includes naturally occurring Clostridial toxin variants, such as, e.g., Clostridial toxin isoforms and Clostridial toxin subtypes; non-naturally occurring Clostridial toxin variants, such as, e.g., conservative Clostridial toxin variants, non-conservative Clostridial toxin variants, Clostridial toxin chimeric variants and active Clostridial toxin fragments thereof, or any combination thereof. A Clostridial toxin also includes Clostridial toxin complexes, which refers to a complex comprising a Clostridial toxin and non-toxin associated proteins (NAPs), such as, *e.g.*, a Botulinum toxin complex, a Tetanus toxin complex, a Baratii toxin complex, and a Butyricum toxin complex. Non-limiting examples of Clostridial toxin complexes include those produced by a *Clostridium botulinum*, such as, *e.g.*, a 900-kDa BoNT/A complex, a 500-kDa BoNT/A complex, a 300-kDa BoNT/A complex, a 500-kDa BoNT/B complex, a 500-kDa BoNT/C₁ complex, a 500-kDa BoNT/D complex, a 300-kDa BoNT/D complex, a 300-kDa BoNT/E complex, and a 300-kDa BoNT/F complex.

"Effective amount" as applied to the biologically active ingredient means that amount of the ingredient which is generally sufficient to affect a desired change in the subject. For example, where the desired effect is a reduction of scar formation, an effective amount of the ingredient is that amount which causes at least a substantial reduction of scar formation, and without resulting in significant toxicity. In other aspects of this embodiment, a therapeutically effective concentration of a Clostridial toxin active ingredient reduces a symptom associated with the aliment being treated by, e.g., at most 10%, at most 20%, at most 30%, at most 40%, at most 50%, at most 60%, at most 70%, at most 80%, at most 90% or at most 100%.

"Fast-acting" as used herein refers to a botulinum toxin that produces effects in the patient more rapidly than those produced by, for example, a botulinum neurotoxin type A, such as onabotulinumtoxinA. For example, the effects of a fast-acting botulinum toxin can be produced within 12 hours, 24 hours or 36 hours. Thus, relative to a fast-acting toxin such as type E, botulinum toxin type A can be classified as a "slower-acting" botulinum toxin. In some cases, a slower-acting botulinum toxin is referred to as an "intermediate-acting" toxin. In some embodiments, fast-acting botulinum toxin produces a measurable therapeutic effect within 6 hours, 12 hours, 24 hours or 36 hours after its administration, and/or its effect is observed at least about 50% sooner than the therapeutic effect produced by onabotulinumtoxinA.

"Fast-recovery" as used herein refers to a botulinum toxin whose effects diminish in a patient more rapidly than those produced by, for example, a botulinum neurotoxin type A, such as onabotulinumtoxinA. In some embodiments, the therapeutic effect of the fast-recovery botulinum toxin diminishes within about 3 months, 2 months or 6 weeks after its administration, and its effects diminish about 50% sooner than the effects produced by onabotulinumtoxinA. For example, the effects of a fast-recovery botulinum toxin can diminish within, for example, 120 hours, 150 hours, 300 hours, 350 hours, 400 hours, 500 hours, 600 hours, 700 hours, 800 hours, or the like. It is known that botulinum toxin type A can have an efficacy for up to 12 months (European J. Neurology 6 (Supp 4): S111-S1150:1999), and in some circumstances for as long as 27 months, when used to treat glands, such as in the treatment of hyperhydrosis. See e.g. Bushara K., Botulinum toxin and rhinorrhea, Otolaryngol Head Neck Surg 1996; 114(3):507, and The Laryngoscope 109:1344-1346:1999. However, the usual duration of an intramuscular injection of a botulinum neurotoxin type A is typically about 3 to 4 months. Thus, relative to a fast-recovery toxin such as type E, botulinum toxin type A, such as onabotulinumtoxinA, can be classified as a "slower-recovery" or "longer acting" botulinum toxin.

"Intermediate-acting" as used herein refers to a botulinum toxin that produces effects more slowly than would a fast-acting toxin.

"Local administration" means direct administration of a pharmaceutical at or to the vicinity of a site on or within an animal body, at which site a biological effect of the pharmaceutical is desired, such as via, for example, intramuscular or intra- or subdermal injection or topical administration. Local administration excludes systemic routes of administration, such as intravenous or oral administration. Topical administration is a type of local administration in which a pharmaceutical agent is applied to a patient's.

"Neurotoxin" means a biologically active molecule with a specific affinity for a neuronal cell surface receptor. Neurotoxin includes Clostridial toxins both as pure toxin and as complexed with one to more non-toxin, toxin associated proteins.

"Patient" means a human or non-human subject receiving medical or veterinary care.

"Pharmaceutical composition" means a composition comprising an active pharmaceutical ingredient, such as, for example, a Clostridial toxin active ingredient such as a botulinum toxin, and at least one additional ingredient, such as, for example, a stabilizer or excipient or the like. A pharmaceutical composition is therefore a formulation which is suitable for diagnostic or therapeutic administration to a subject, such as a human patient. The pharmaceutical composition can be, for example, in a lyophilized or vacuum dried condition, a solution formed after reconstitution of the lyophilized or vacuum dried pharmaceutical composition, or as a solution or solid which does not require reconstitution. As stated, a pharmaceutical composition can be liquid or solid. A pharmaceutical composition can be animal-protein free.

"Preparing" a surgical site refers to administering a composition disclosed herein to reduce muscle tension in the incision area.

"Supplemental administration" as used herein refers to a botulinum administration that follows an initial neurotoxin administration.

"Therapeutic formulation" means a formulation that can be used to treat and thereby alleviate a disorder, or a disease and/or symptom associated thereof, such as a disorder or a disease characterized by an activity of a peripheral muscle.

"Therapeutically effective concentration", "therapeutically effective amount," "effective amount," "effective dose," and "therapeutically effective dose" refer to the minimum dose of an agent (e.g. such as a botulinum toxin or pharmaceutical composition comprising botulinum toxin) needed to achieve the desired therapeutic effect and includes a dose sufficient to reduce a symptom associated with a disease, disorder or condition being treated without causing significant negative or adverse side effects.

"Treat," "treating," or "treatment" means an alleviation or a reduction (which includes some reduction, a significant reduction a near total reduction, and a total reduction), resolution or prevention (temporarily or permanently) of an disease, disorder or condition, so as to achieve a desired therapeutic or cosmetic result, such as by healing of injured or damaged tissue, or by altering, changing, enhancing, improving, ameliorating and/or beautifying an existing or perceived disease, disorder or condition.

"Unit" or "U" means an amount of active botulinum neurotoxin standardized to have equivalent neuromuscular blocking effect as a Unit of commercially available botulinum neurotoxin type A.

"Wound" as used herein refers to a disruption to the skin, for example caused by injury or intentionally.

Groupings of alternative embodiments, elements, or steps of the present disclosure are not to be construed as limitations. Each group member may be referred to and claimed individually or in any combination with other group members disclosed herein. It is anticipated that one or more members of a group may be comprised in, or deleted from, a group for reasons of convenience and/or patentability. When any such inclusion or deletion occurs, the specification is deemed to contain the group as modified thus fulfilling the written description of all Markush groups used in the appended claims.

Unless otherwise indicated, all numbers expressing a characteristic, item, quantity, parameter, property, term, and so forth used in the present specification and claims are to be understood as being modified in all instances by the term "about." As used herein, the term "about" means that the characteristic, item, quantity, parameter, property, or term so qualified encompasses a range of plus or minus ten percent above and below the value of the stated characteristic, item, quantity, parameter, property, or term. Accordingly, unless indicated to the contrary, the numerical parameters set forth in the specification and attached claims are approximations that may vary. At the very least, and not as an attempt to limit the application of the doctrine of equivalents to the scope of the claims, each numerical indication should at least be construed in light of the number of reported significant digits and by applying ordinary rounding techniques. Notwithstanding that the numerical ranges and values setting forth the broad scope of the disclosure are approximations, the numerical ranges and values set forth in the specific examples are reported as precisely as possible. Any numerical range or value, however, inherently contains certain errors necessarily resulting from the standard deviation found in their respective testing measurements. Recitation of numerical ranges of values herein is merely intended to serve as a shorthand method of referring individually to each separate numerical value falling within the range. Unless otherwise indicated herein, each individual value of a numerical range is incorporated into the present specification as if it were individually recited herein.

The terms "a," "an," "the" and similar referents used in the context of describing the disclosure (especially in the context of the following claims) are to be construed to cover both the singular and the plural, unless otherwise indicated herein or clearly contradicted by context. All methods described herein can be performed in any suitable order unless otherwise indicated herein or otherwise clearly contradicted by context. The use of any and all examples, or exemplary language (e.g., "such as") provided herein is intended merely to better illuminate the disclosure and does not pose a limitation on the scope otherwise claimed. No language in the present specification should be construed as indicating any non-claimed element essential to the practice of embodiments disclosed herein.

### Neurotoxin Compositions

Embodiments disclosed herein comprise neurotoxin compositions, for example fast-recovery neurotoxins. Such neurotoxins can be formulated in any pharmaceutically acceptable formulation in any pharmaceutically acceptable form. The neurotoxin can also be used in any pharmaceutically acceptable form supplied by any manufacturer.

The neurotoxin can be made by a *Clostridial* bacterium, such as by a *Clostridium botulinum*, *Clostridium butyricum*, or Clostridium *beratti bacterium.* Additionally, the neurotoxin can be a modified neurotoxin; that is a neurotoxin that has at least one of its amino acids deleted, modified or replaced, as compared to the native or wild type neurotoxin. Furthermore, the neurotoxin can be a recombinantly produced neurotoxin or a derivative or fragment thereof.

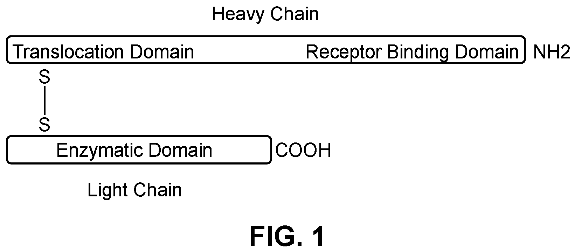

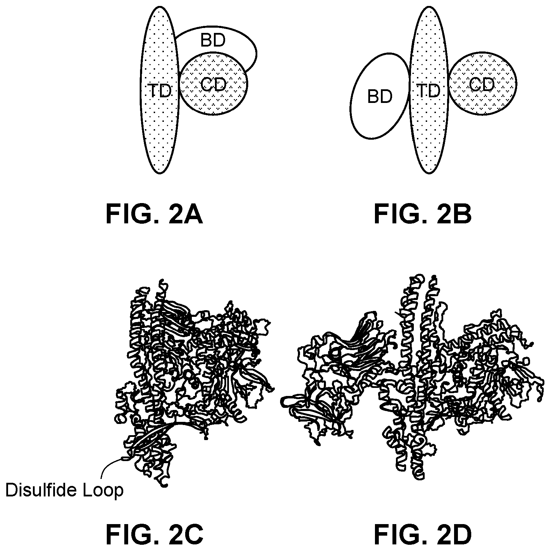

**FIG. 1** depicts the primary structure of a botulinum neurotoxin (BoNT). **FIGS. 2A-2D** are schematic and ribbon representations of BoNT/E (FIGS. 2A, 2C) and BoNT/B (FIGS. 2B, 2D). The catalytic, translocation, and binding domains are labeled CD, TD, and BD, respectively.

In some embodiments, a disclosed botulinum neurotoxin type E (BoNT/E) composition has 40% amino acid homology compared with type A and they share the same basic domain structure consisting of 2 chains, a 100 kDa heavy chain (HC) and a 50 kDa light chain (LC), linked by a disulfide bond, see e.g. Whelan et al., The complete amino acid sequence of the Clostridium botulinum type-E neurotoxin, derived by nucleotide-sequence analysis of the encoding gene, Eur. J. Biochem. 204(2): 657-667 (1992). The HC contains the receptor binding domain and the translocation domain while the LC contains the synaptosomal-associated protein (SNAP) enzymatic activity. The domain structure is the same structure shared by all botulinum neurotoxin serotypes.

In disclosed embodiments, the neurotoxin is formulated in unit dosage form; for example, it can be provided as a sterile solution in a vial or as a vial or sachet containing a lyophilized powder for reconstituting a suitable vehicle such as saline for injection.

In some embodiments, the botulinum toxin is formulated in a solution containing saline and pasteurized human serum albumin, which stabilizes the toxin and minimizes loss through nonspecific adsorption. The solution can be sterile filtered (0.2 µm filter), filled into individual vials and then vacuum-dried to give a sterile lyophilized powder. In use, the powder can be reconstituted by the addition of sterile unpreserved normal saline (sodium chloride 0.9% for injection).

In an embodiment, botulinum type E is supplied in a sterile solution for injection with a 5-mL vial nominal concentration of 20 ng/mL in 0.03 M sodium phosphate, 0.12 M sodium chloride, and 1 mg/mL Human Serum Albumin (HSA), at pH 6.0. In another embodiment, the botulinum type E is provided as a liquid formulation comprised of sodium phosphate, sodium chloride, and HSA. In yet another embodiment, the botulinum type E is supplied as a lyophilized or liquid formulation comprising a surfactant and a disaccharide.

Although the composition may only contain a single type of neurotoxin, for example botulinum type E, disclosed compositions can include two or more types of neurotoxins, which can provide enhanced therapeutic effects of the disorders. For example, a composition administered to a patient can include botulinum types A and E. Administering a single composition containing two different neurotoxins can permit the effective concentration of each of the neurotoxins to be lower than if a single neurotoxin is administered to the patient while still achieving the desired therapeutic effects. The composition administered to the patient can also contain other pharmaceutically active ingredients, such as, protein receptor or ion channel modulators, in combination with the neurotoxin or neurotoxins. These modulators may contribute to the reduction in neurotransmission between the various neurons. For example, a composition may contain gamma aminobutyric acid (GABA) type A receptor modulators that enhance the inhibitory effects mediated by the GABA_{A} receptor. The GABA_{A} receptor inhibits neuronal activity by effectively shunting current flow across the cell membrane. GABA_{A} receptor modulators may enhance the inhibitory effects of the GABA_{A} receptor and reduce electrical or chemical signal transmission from the neurons. Examples of GABA_{A} receptor modulators include benzodiazepines, such as diazepam, oxaxepam, lorazepam, prazepam, alprazolam, halazeapam, chordiazepoxide, and chlorazepate. Compositions may also contain glutamate receptor modulators that decrease the excitatory effects mediated by glutamate receptors. Examples of glutamate receptor modulators include agents that inhibit current flux through AMPA, NMDA, and/or kainate types of glutamate receptors.

### Methods of Treatment

Methods disclosed herein can comprise administration of a fast-acting neurotoxin to a patient. In a preferred embodiment the neurotoxin is botulinum type E.

Methods disclosed herein can comprise supplemental administration of a fast-acting neurotoxin to a patient. Embodiments comprising supplemental administration can further comprise doctor or patient evaluation of the results of a prior neurotoxin administration.

In some embodiments, administration of the fast-acting neurotoxin is performed prior to a surgical procedure. In some embodiments, the administration is performed, for example, within 6 hours before the procedure, within 5 hours before the procedure, within 4 hours before the procedure, within 3 hours before the procedure, within 2 hours before the procedure, within 60 minutes before the procedure, within 50 minutes before the procedure, within 40 minutes before the procedure, within 30 minutes before the procedure, within 20 minutes before the procedure, within 10 minutes before the procedure, within 5 minutes before the procedure, within 2 minutes before the procedure, or the like.

In alternative embodiments, administration of the fast-acting neurotoxin is performed concurrently with a surgical procedure.

In other embodiments, administration of the fast-acting neurotoxin is performed after a surgical procedure. For example, administration can be performed, within 1 minute after the procedure, within 2 minutes after the procedure, within 3 minutes after the procedure, within 4 minutes after the procedure, within 5 minutes after the procedure, within 6 minutes after the procedure, within 7 minutes after the procedure, within 8 minutes after the procedure, within 9 minutes after the procedure, within 10 minutes after the procedure, within 20 minutes after the procedure, within 30 minutes after the procedure, within 40 minutes after the procedure, within 50 minutes after the procedure, within 60 minutes after the procedure, within 90 minutes after the procedure, within 120 minutes after the procedure, within 180 minutes after the procedure, within 240 minutes after the procedure, within 300 minutes after the procedure, or the like. In some other embodiments, administration can be performed within 1 to 3 days after the procedure. In yet other embodiments, administration can be performed within 3 months after the procedure.

In embodiments comprising a supplemental administration, evaluation of the results of the initial neurotoxin administration can be performed within, for example, 6 hours of the initial administration, 8 hours of the initial administration, 10 hours of the initial administration, 12 hours of the initial administration, 14 hours of the initial administration, 16 hours of the initial administration, 18 hours of the initial administration, 24 hours of the initial administration, 30 hours of the initial administration, 36 hours of the initial administration, 42 hours of the initial administration, 48 hours of the initial administration, 54 hours of the initial administration, 60 hours of the initial administration, 66 hours of the initial administration, 72 hours of the initial administration, 78 hours of the initial administration, 84 hours of the initial administration, 90 hours of the initial administration, 96 hours of the initial administration, 102 hours of the initial administration, 108 hours of the initial administration, 114 hours of the initial administration, 120 hours of the initial administration, 1 week of the initial administration, 2 weeks of the initial administration, 3 weeks of the initial administration, 4 weeks of the initial administration, 5 weeks of the initial administration, 6 weeks of the initial administration, 7 weeks of the initial administration, 8 weeks of the initial administration, 9 weeks of the initial administration, 10 weeks of the initial administration, 11 weeks of the initial administration, 12 weeks of the initial administration, or the like.

In embodiments comprising a supplemental administration, administration of the supplemental dose can be performed, within, for example, 6 hours of the evaluation, 8 hours of the evaluation, 10 hours of the evaluation, 12 hours of the evaluation, 14 hours of the evaluation, 16 hours of the evaluation, 18 hours of the evaluation, 24 hours of the evaluation, 30 hours of the evaluation, 36 hours of the evaluation, 42 hours of the evaluation, 48 hours of the evaluation, 54 hours of the evaluation, 60 hours of the evaluation, 66 hours of the evaluation, 72 hours of the evaluation, 78 hours of the evaluation, 84 hours of the evaluation, 90 hours of the evaluation, 96 hours of the evaluation, 102 hours of the evaluation, 108 hours of the evaluation, 114 hours of the evaluation, 120 hours of the evaluation, 1 week of the evaluation, 2 weeks of the evaluation, 3 weeks of the evaluation, 4 weeks of the evaluation, 5 weeks of the evaluation, 6 weeks of the evaluation, 7 weeks of the evaluation, 8 weeks of the evaluation, 9 weeks of the evaluation, 10 weeks of the evaluation, 11 weeks of the evaluation, 12 weeks of the evaluation, or the like.

In some embodiments, the supplemental administration itself can be performed, for example, within, for example, 6 hours of the initial administration of the fast-acting neurotoxin, 8 hours of the initial administration, 10 hours of the initial administration, 12 hours of the initial administration, 14 hours of the initial administration, 16 hours of the initial administration, 18 hours of the initial administration, 24 hours of the initial administration, 30 hours of the initial administration, 36 hours of the initial administration, 42 hours of the initial administration, 48 hours of the initial administration, 54 hours of the initial administration, 60 hours of the initial administration, 66 hours of the initial administration, 72 hours of the initial administration, 78 hours of the initial administration, 84 hours of the initial administration, 90 hours of the initial administration, 96 hours of the initial administration, 102 hours of the initial administration, 108 hours of the initial administration, 114 hours of the initial administration, 120 hours of the initial administration, 1 week of the initial administration, 2 weeks of the initial administration, 3 weeks of the initial administration, 4 weeks of the initial administration, 5 weeks of the initial administration, 6 weeks of the initial administration, 7 weeks of the initial administration, 8 weeks of the initial administration, 9 weeks of the initial administration, 10 weeks of the initial administration, 11 weeks of the initial administration, 12 weeks of the initial administration, or the like. In some embodiments, the supplemental administration can be performed, for example, within, for example, 3 to 6 months of the initial administration. In some embodiments, the supplemental administration can be performed concurrently as the initial administration.

Methods disclosed herein can provide rapid-onset effects (for example, using a fast-acting neurotoxin). For example, disclosed embodiments can reduce muscle activity in the proximity of a surgical incision within, for example, 30 minutes after administration, 45 minutes after administration, 60 minutes after administration, 75 minutes after administration, 90 minutes after administration, 2 hours after administration, 3 hours after administration, 4 hours after administration, 5 hours after administration, 6 hours after administration, 7 hours after administration, 8 hours after administration, 9 hours after administration, 10 hours after administration, 11 hours after administration, 12 hours after administration, 13 hours after administration, 14 hours after administration, 15 hours after administration, 16 hours after administration, 17 hours after administration, 18 hours after administration, 19 hours after administration, 20 hours after administration, 21 hours after administration, 22 hours after administration, 23 hours after administration, 24 hours after administration, 30 hours after administration, 36 hours after administration, 42 hours after administration, 48 hours after administration, 3 days after administration, 4 days after administration, 5 days after administration, 6 days after administration, 7 days after administration, or the like.

Methods disclosed herein can provide reduction in muscle activity for a shorter duration (for example, using a fast-recovery neurotoxin). For example, disclosed embodiments can provide a reduction in muscle activity that subsides within, for example, 3 days after administration, 4 days after administration, 5 days after administration, 6 days after administration, 7 days after administration, 8 days after administration, 9 days after administration, 10 days after administration, 11 days after administration, 12 days after administration, 13 days after administration, 14 days after administration, 15 days after administration, 16 days after administration, 17 days after administration, 18 days after administration, 19 days after administration, 20 days after administration, 21 days after administration, 22 days after administration, 23 days after administration, 24 days after administration, 25 days after administration, 26 days after administration, 27 days after administration, 28 days after administration, 29 days after administration, 30 days after administration, 45 days after administration, 60 days after administration, 75 days after administration, 90 days after administration, 105 days after administration, or the like.

Side-effects can be associated with botulinum injections. Disclosed embodiments can provide neurotoxin treatments that result in fewer side effects, or side effects of a shortened duration, than conventional neurotoxin treatments, for example treatment using a longer acting neurotoxin such as type A.

For example, disclosed embodiments can result in fewer (or shorter duration) instances of double vision or blurred vision, eyelid paralysis (subject cannot lift eyelid all the way open), loss of facial muscle movement, hoarseness, loss of bladder control, shortness of breath, difficulty in swallowing, difficulty speaking, death, and the like.

The disclosed methods comprise administration to an area prone to scarring, for example an area in the proximity of a surgical incision, or an area in the proximity of any injury to the skin, for example a traumatic injury. Disclosed embodiments comprise administration to muscles proximate to an area prone to scarring, for example, to skeletal muscle tissue or smooth muscle tissue.

Further, disclosed embodiments can provide reduced muscle activity of a more- certain duration. For example, with a longer acting neurotoxin, a 20% variance in duration of effects can result in a month's difference in effective duration. With the disclosed fast-recovery neurotoxins, this 20% variance produces a much less drastic difference in effective duration.

Disclosed fast-acting neurotoxin compositions can be injected into the individual using a needle or a needleless device. In certain embodiments, the method comprises subdermally injecting the composition in the individual. For example, the administering may comprise injecting the composition through a needle no greater than about 30 gauge. In certain embodiments, the method comprises administering a composition comprising a botulinum toxin type E.

Injection of the compositions can be carried out by syringe, catheters, needles and other means for injecting. The injection can be performed on any area of the mammal's body that is in need of treatment, including, but not limited to, face, neck, torso, arms, hands, legs, and feet. The injection can be into any position in the specific area such as epidermis, dermis, fat, muscle, or subcutaneous layer.

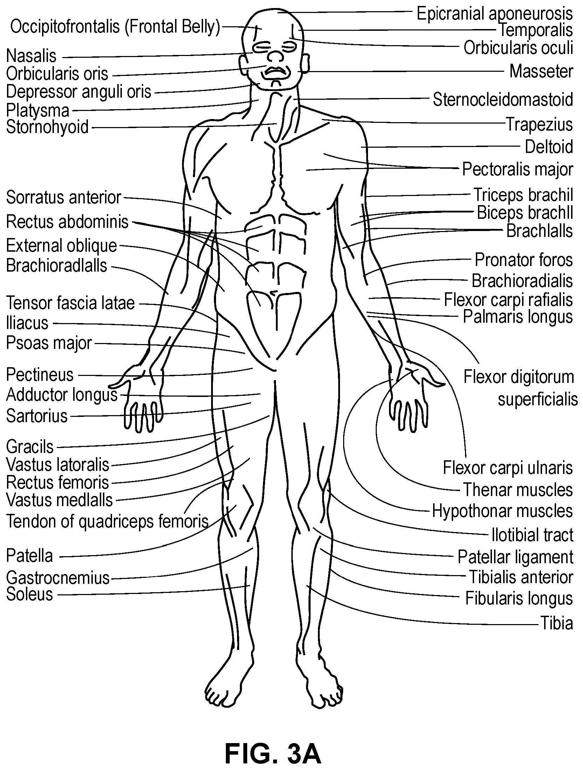

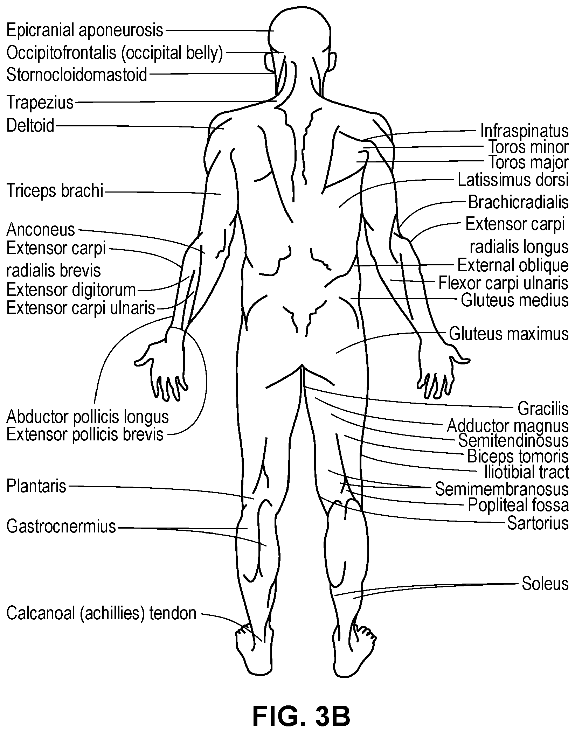

For example, skeletal muscles suitable for administration of disclosed compositions can comprise any of the muscles, or combinations of muscles, of the illustrations shown in **FIGS. 3A-****3B.**

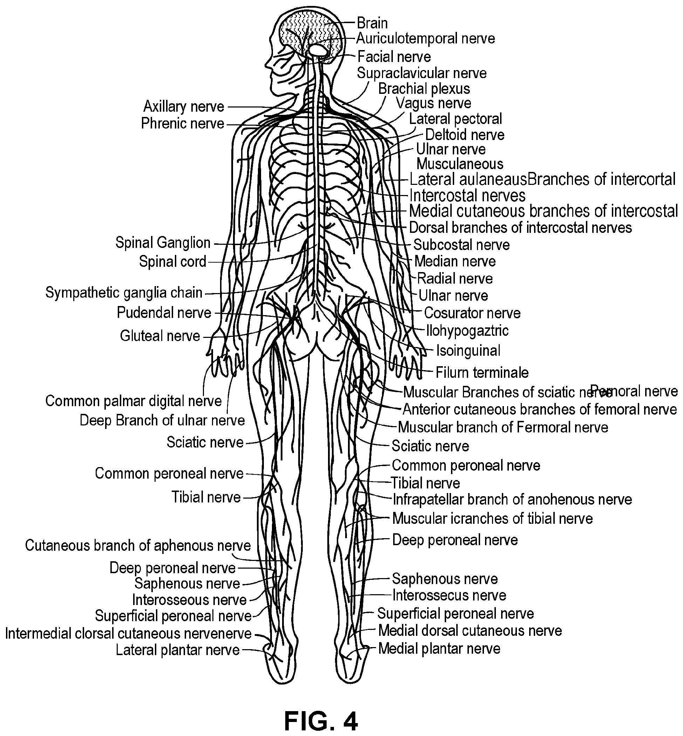

Administration can comprise injection into or in the vicinity of one or more of the nerves shown in the schematic of **FIG. 4****.**

Smooth muscles suitable for administration of disclosed compositions can comprise any of walls of blood vessels, walls of stomach, ureters, intestines, in the aorta (tunica media layer), iris of the eye, prostate, gastrointestinal tract, respiratory tract, small arteries, arterioles, reproductive tracts (both genders), veins, glomeruli of the kidneys (called mesangial cells), bladder, uterus, arrector pili of the skin, ciliary muscle, sphincter, trachea, bile ducts, and the like.

The frequency and the amount of injection under the disclosed methods can be determined based on the nature and location of the particular area being treated. In certain cases, however, repeated injection may be desired to achieve optimal results. The frequency and the amount of the injection for each particular case can be determined by the person of ordinary skill in the art.

Although examples of routes of administration and dosages are provided, the appropriate route of administration and dosage are generally determined on a case by case basis by the attending physician. Such determinations are routine to one of ordinary skill in the art (see for example, Harrison's Principles of Internal Medicine (1998), edited by Anthony Fauci et al., 14th edition, published by McGraw Hill). For example, the route and dosage for administration of a Clostridial neurotoxin according to the present disclosed invention can be selected based upon criteria such as the solubility characteristics of the neurotoxin chosen as well as the intensity and scope of the condition being treated.

The fast-acting neurotoxin can be administered in an amount of between about 10⁻³ U/kg and about 35 U/kg. In an embodiment, the neurotoxin is administered in an amount of between about 10-² U/kg and about 25 U/kg. In another embodiment, the neurotoxin is administered in an amount of between about 10⁻¹ U/kg and about 15 U/kg. In another embodiment, the neurotoxin is administered in an amount of between about 1 U/kg and about 10 U/kg. In many instances, an administration of from about 1 unit to about 500 units of a neurotoxin, such as a botulinum type E, provides effective therapeutic relief. In an embodiment, from about 5 units to about 200 units of a neurotoxin, such as a botulinum type E, can be used and in another embodiment, from about 10 units to about 100 units of a neurotoxin, such as a botulinum type E, can be locally administered into a target tissue such as a muscle.

In some embodiments, administration can comprise a dose of about 10 units of a neurotoxin, or about 20 units of a neurotoxin, or about 30 units of a neurotoxin, or about 40 units of a neurotoxin, or about 50 units of a neurotoxin, or about 60 units of a neurotoxin, or about 70 units of a neurotoxin, or about 80 units of a neurotoxin, or about 90 units of a neurotoxin, or about 100 units of a neurotoxin, or about 110 units of a neurotoxin, or about 120 units of a neurotoxin, or about 130 units of a neurotoxin, or about 140 units of a neurotoxin, or about 150 units of a neurotoxin, or about 160 units of a neurotoxin, or about 170 units of a neurotoxin, or about 180 units of a neurotoxin, or about 190 units of a neurotoxin, or about 200 units of a neurotoxin, or about 210 units of a neurotoxin, or about 220 units of a neurotoxin, or about 230 units of a neurotoxin, or about 240 units of a neurotoxin, or about 250 units of a neurotoxin, or about 260 units of a neurotoxin, or about 270 units of a neurotoxin, or about 280 units of a neurotoxin, or about 290 units of a neurotoxin, or about 290 units of a neurotoxin, or about 300 units of a neurotoxin, or about 310 units of a neurotoxin, or about 320 units of a neurotoxin, or about 330 units of a neurotoxin, or about 340 units of a neurotoxin, or about 350 units of a neurotoxin, or about 360 units of a neurotoxin, or about 370 units of a neurotoxin, or about 380 units of a neurotoxin, or about 390 units of a neurotoxin, or about 400 units of a neurotoxin, or about 410 units of a neurotoxin, or about 420 units of a neurotoxin, or about 430 units of a neurotoxin, or about 440 units of a neurotoxin, or about 450 units of a neurotoxin, or about 460 units of a neurotoxin, or about 470 units of a neurotoxin, or about 480 units of a neurotoxin, or about 490 units of a neurotoxin, or about 500 units of a neurotoxin, or the like.

In some embodiments, administration can comprise a dose of about 0.1 nanograms (ng) of a neurotoxin, 0.2 ng of a neurotoxin, 0.3 ng of a neurotoxin, 0.4 ng of a neurotoxin, 0.5 ng of a neurotoxin, 0.6 ng of a neurotoxin, 0.7 ng of a neurotoxin, 0.8 ng of a neurotoxin, 0.9 ng of a neurotoxin, 1.0 ng of a neurotoxin, 1.1 ng of a neurotoxin, 1.2 ng of a neurotoxin, 1.3 ng of a neurotoxin, 1.4 ng of a neurotoxin, 1.5 ng of a neurotoxin, 1.6 ng of a neurotoxin, 1.7 ng of a neurotoxin, 1.8 ng of a neurotoxin, 1.9 ng of a neurotoxin, 2.0 ng of a neurotoxin, 2.1 ng of a neurotoxin, 2.2 ng of a neurotoxin, 2.3 ng of a neurotoxin, 2.4 ng of a neurotoxin, 2.5 ng of a neurotoxin, 2.6 ng of a neurotoxin, 2.7 ng of a neurotoxin, 2.8 ng of a neurotoxin, 2.9 ng of a neurotoxin, 3.0 ng of a neurotoxin, 3.1 ng of a neurotoxin, 3.2 ng of a neurotoxin, 3.3 n of a neurotoxin, 3.4 ng of a neurotoxin, 3.5 ng of a neurotoxin, 3.6 ng of a neurotoxin, 3.7 ng of a neurotoxin, 3.8 ng of a neurotoxin, 3.9 ng of a neurotoxin, 4.0 ng of a neurotoxin, 4.1 ng of a neurotoxin, 4.2 ng of a neurotoxin, 4.3 ng of a neurotoxin, 4.4 ng of a neurotoxin, 4.5 ng of a neurotoxin, 5 ng of a neurotoxin, 6 ng of a neurotoxin, 7 ng of a neurotoxin, 8 ng of a neurotoxin, 9 ng of a neurotoxin, 10 ng of a neurotoxin, or the like.

In some embodiments, administration can comprise a dose of between about 0.1 nanograms (ng) of a neurotoxin and 20 ng of a neurotoxin, between about 1 ng of a neurotoxin and 19 ng of a neurotoxin, between about 2 ng of a neurotoxin and 18 ng of a neurotoxin, between about 3 ng of a neurotoxin and 17 ng of a neurotoxin, between about 4 ng of a neurotoxin and 16 ng of a neurotoxin, between about 5 ng of a neurotoxin and 15 ng of a neurotoxin, between about 6 ng of a neurotoxin and 14 ng of a neurotoxin, between about 7 ng of a neurotoxin and 13 ng of a neurotoxin, between about 8 ng of a neurotoxin and 12 ng of a neurotoxin, between about 9 ng of a neurotoxin and 11 ng of a neurotoxin, or the like. In embodiments, administration can comprise one or more injections of about 0.1 nanograms (ng) of a neurotoxin, 0.2 ng of a neurotoxin, 0.3 ng of a neurotoxin, 0.4 ng of a neurotoxin, 0.5 ng of a neurotoxin, 0.6 ng of a neurotoxin, 0.7 ng of a neurotoxin, 0.8 ng of a neurotoxin, 0.9 ng of a neurotoxin, 1.0 ng of a neurotoxin, 1.1 ng of a neurotoxin, 1.2 ng of a neurotoxin, 1.3 ng of a neurotoxin, 1.4 ng of a neurotoxin, 1.5 ng of a neurotoxin, 1.6 ng of a neurotoxin, 1.7 ng of a neurotoxin, 1.8 ng of a neurotoxin, 1.9 ng of a neurotoxin, 2.0 ng of a neurotoxin, 2.1 ng of a neurotoxin, 2.2 ng of a neurotoxin, 2.3 ng of a neurotoxin, 2.4 ng of a neurotoxin, 2.5 ng of a neurotoxin, 2.6 ng of a neurotoxin, 2.7 ng of a neurotoxin, 2.8 ng of a neurotoxin, 2.9 ng of a neurotoxin, 3.0 ng of a neurotoxin, 3.1 ng of a neurotoxin, 3.2 ng of a neurotoxin, 3.3 n of a neurotoxin, 3.4 ng of a neurotoxin, 3.5 ng of a neurotoxin, 3.6 ng of a neurotoxin, 3.7 ng of a neurotoxin, 3.8 ng of a neurotoxin, 3.9 ng of a neurotoxin, 4.0 ng of a neurotoxin, 4.1 ng of a neurotoxin, 4.2 ng of a neurotoxin, 4.3 ng of a neurotoxin, 4.4 ng of a neurotoxin, 4.5 ng of a neurotoxin, 5 ng of a neurotoxin, 6 ng of a neurotoxin, 7 ng of a neurotoxin, 8 ng of a neurotoxin, 9 ng of a neurotoxin, 10 ng of a neurotoxin, or the like.

In some embodiments, administration can comprise one or more injections of between about 0.1 nanograms (ng) of a neurotoxin and 20 ng of a neurotoxin, between about 1 ng of a neurotoxin and 19 ng of a neurotoxin, between about 2 ng of a neurotoxin and 18 ng of a neurotoxin, between about 3 ng of a neurotoxin and 17 ng of a neurotoxin, between about 4 ng of a neurotoxin and 16 ng of a neurotoxin, between about 5 ng of a neurotoxin and 15 ng of a neurotoxin, between about 6 ng of a neurotoxin and 14 ng of a neurotoxin, between about 7 ng of a neurotoxin and 13 ng of a neurotoxin, between about 8 ng of a neurotoxin and 12 ng of a neurotoxin, between about 9 ng of a neurotoxin and 11 ng of a neurotoxin, or the like.

Ultimately, however, both the quantity of toxin administered and the frequency of its administration will be at the discretion of the physician responsible for the treatment and will be commensurate with questions of safety and the effects produced by the toxin.

In some embodiments, administration can comprise one or more injections, for example injections substantially along the incision site or line or lines. In some embodiments, administration can comprise injections in a specific pattern, for example, a W pattern, and X patter, a Z pattern, a star pattern, a circle pattern, a half circle pattern, a square pattern, a rectangle pattern, a crescent patter, or combinations thereof.

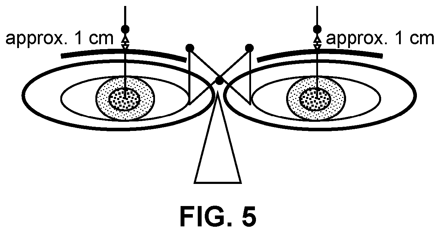

A study was conducted where BoNT/E was administered to human patients undergoing treatment of glabellar lines, in order to evaluate onset of action, safety and tolerability of BoNT/E in humans. The study is described in Example 1. The BoNT/E was administered into the procerus muscle at five injection points, as illustrated in **FIG. 5****.** BoNT/E provided improvement in severity of glabellar lines with fast onset of action, and with a favorable safety and tolerability profile.

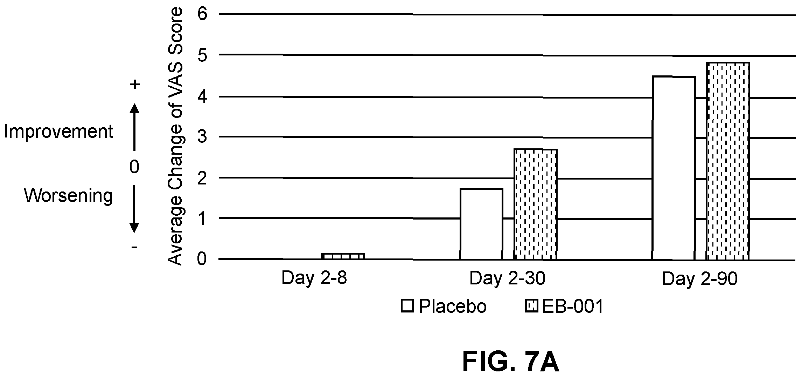

Another study was conducted where BoNT/E was administered to human patients undergoing surgical treatment of basal cell carcinoma. As described in Example 5, twelve human subjects scheduled for Mohs surgery to remove a single lesion of basal cell carcinoma on the forehead were randomized into a treatment group (n=8) and a placebo group (n=4). The subjects in the both groups received a single intramuscular injection in the frontalis muscle near the surgical site, the treatment group receiving a dose of BoNT/E and the placebo group receiving saline. Beginning on Day 2 of the study (24 hours post-op), each subject was assessed using the VAS, SCAR and POSAS scales (see Example 4). The subjects were again assessed using the various scales on days 8, 30 and 90 of the study. Results are shown in **FIGS. 7A-7C****,** where the time point along the x-axis in each bar graph denoted as Day x-y is calculated as the change in assessment score on Day y - Day x.

**FIG. 7A** is a bar graph showing the average change of investigator rated Visual Analog Scale (VAS) on day 2-8, day 2-30 and day 2-90. The VAS is a 0 - 10 numeric scale, where 0 is the worst possible scar and 10 is the best possible scar. In the graph, the bars with dashed fill correspond to the patients treated with BoNT/E intramuscularly in the frontalis muscle during Mohs surgery the open bars (no fill) correspond to the patients in the placebo group treated with saline. The subjects treated with BoNT/E had a larger average change of VAS score for the assessments conducted on day 2 and day 30, during the acute scar formation phase during surgical wound healing, relative to the subjects not treated with BoNT/E.

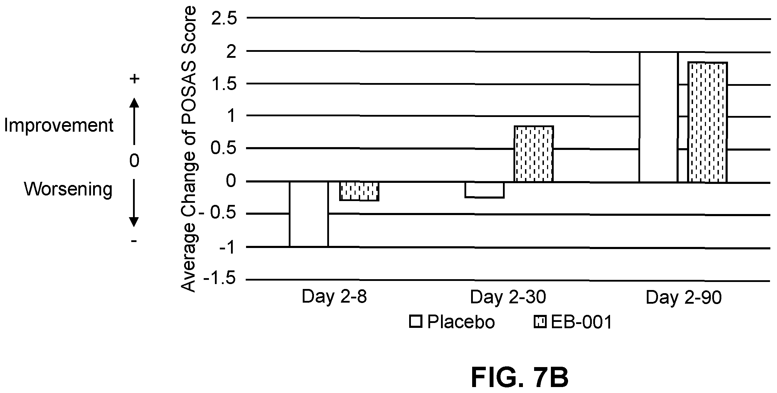

Using the Patient and Observer Scar Assessment Scale (POSAS), the patients assessed the color of their scars on days 2, 8, 30 and 90. The color component is a 1 to 10 scale, where a score of 1 corresponds to the color of the subject's normal skin and a score of 10 corresponds to a 'very different' color of the scar relative to the subject's normal skin. The bar graph in **FIG. 7B** shows that administration of BoNT/E intramuscularly in the frontalis muscle during Mohs surgery improves the discoloration observed by the patients during the first 30 days following surgery.

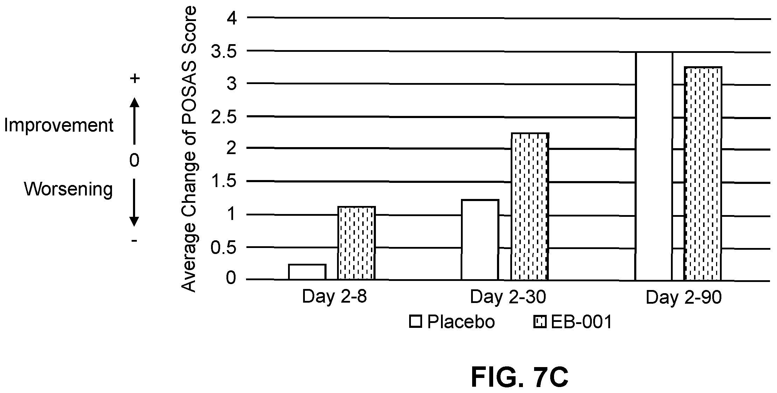

The patients in the study also used the POSAS to assess stiffness of the tissue at the wound site. Using a 1 to 10 scale, where a score of 1 corresponds to the stiffness of the subject's normal skin and a score of 10 corresponds to a 'very different' stiffness relative to the subject's normal skin, the patients did an assessment on days 2, 8, 30 and 90. **FIG. 7C** is a bar graph showing the average change of patient-reported stiffness component of scar assessment using the Patient and Observer Scar Assessment Scale (POSAS) on day 2-8, day 2-30 and day 2-90. The patients treated with BoNT/E intramuscularly in the frontalis muscle during Mohs surgery (bars with dashed fill) reported improved physical characteristics of the scar compared to the patients not treated with botulinum toxin as part of a Mohs surgery (placebo, open bars).

The subjects in the study of Example 5 were questioned about itch and pain associated with the scar. The subjects were asked on days 2, 8, 30 and 90: "Have you been bothered by any itch from the scar in the past 24 hours?". The subjects were also asked on days 2, 8, 30 and 90 "Have you been bothered by any pain from the scar in the past 24 hours?". A summary of the subjects who responded affirmatively to the questions is in the tables below.
Number and Percent of Subjects Responding "Yes" to question regarding itch

| Group | Day 2 | Day 8 | Day 30 | Day 90 |
|---|---|---|---|---|
| Placebo (n=4) | 3 (75%) | 2 (50%) | 0 | 0 |
| BoNT/E (n=8) | 0 | 0 | 0 | 0 |

Number and Percent of Subjects Responding "Yes" to question regarding pain

| Group | Day 2 | Day 8 | Day 30 | Day 90 |
|---|---|---|---|---|
| Placebo (n=4) | 4 (100%) | 2 (50%) | 0 | 0 |
| BoNT/E (n=8) | 6 (75%) | 2 (25%) | 0 | 0 |

Subjects treated with BoNT/E reported a reduction in itch and a reduction in pain associated with the scar during the acute scar formation phase during wound healing beginning after wound creation through about day 21, day 25, or day 30 after wound creation. Accordingly, in one embodiment, a method of minimizing scar formation and improving scar appearance associated with healing of a wound is accompanied by a method of reducing itch and/or pain associated with the scar. That is, treatment of the wound or the muscle(s) underlying the wound with BoNT/E provides an improved scar appearance and reduces itch or pain associated with scar formation (or wound healing). In one embodiment, administration of BoNT/E reduces the sensation of itch for 24-48 hours associated with the scar in 75% or more of the patients receiving BoNT/E treatment. In another embodiment, administration of BoNT/E reduces the pain 2-8 days or for 1 week following wound creation and during wound healing in at least about 25% or 50% of patients receiving BoNT/E treatment.

Photographs of the surgical wound was taken before surgery on Day 1 of the study and on Day 2 (24 hours after surgery) and on Day 30 and Day 90. The photographs (not shown here) reveal that subjects treated with BoNT/E have less redness of the scar at Day 30 and at Day 90 relative to subjects not treated with BoNT/E.

Accordingly, in one aspect, a method for minimizing scarring of a wound is provided by administering botulinum neurotoxin type E toxin in the proximity of the wound. As evidenced by the data of Example 5, BoNT/E administered to a wound or to the muscles in the proximity of the wound improves outcome of scar appearance relative to placebo and gives additional benefits of itch reduction and less pain, particularly during the acute phase of scar formation in the first week or 10 days after wound creation.

In another aspect, a method for minimizing scarring of a wound or a method for improving appearance of a scar is provided. The method comprises administering BoNT/E to or in the proximity of the wound prior to, during or after creation of the wound. Administration of BoNT/E improves appearance of the scar and/or minimizes scar formation, and additionally improves discoloration of the scar within 2-8 days after BoNT/E administration. In one embodiment, the treatment method provides a reduced redness of the scar 30 days, 60 days or 90 days after treatment with BoNT/E. In another embodiment, the treatment method reduces stiffness of the scar during the first 30 days following treatment relative to subjects not treated with BoNT/E.

The study in Example 5 shows that the fast-acting BoNT/E offers a benefit during wound healing as its action has more immediate effect than a slower-acting toxin. A fast-acting toxin present at the wound site in the hours and days subsequent to wound creation when the biology of scar formation is initiated provides a benefit in the overall appearance of the scar, reducing its redness and stiffness and reducing pain and itch. BoNT/E offers a fast onset of action to still or essentially paralyze the muscles in the proximity of the wound in the minutes and hours following its creation, or even, if the BoNT/E is administered prior to wound creation, before its creation. The short duration of the BoNT/E is advantageous as the wound healing process is on the order of weeks to a month, which correlates with the duration of effect of BoNT/E.

In one embodiment, it is contemplated to minimize scarring or improve scar appearance of a scar that forms over a wound created over an existing scar. For example, a subject having a second surgical procedure at a same or similar site as a prior surgical procedure will have a surgical wound created at a site that overlaps with an existing scar from the prior surgery. Scars formed over an existing scar can exhibit more discoloration and/or redness than scars that form over skin. The treatment method herein is contemplated for patients with a wound that is at site that overlaps partially or essentially completely with an existing scar.

A controlled release system can be used in the embodiments described herein to deliver a neurotoxin *in vivo* at a predetermined rate over a specific time period. Generally, release rates are determined by the design of the system and can be largely independent of environmental conditions such as pH. Controlled release systems which can deliver a drug over a period of several years are known. Contrarily, sustained release systems typically deliver drug in 24 hours or less and environmental factors can influence the release rate. Thus, the release rate of a neurotoxin from an implanted controlled release system (an "implant") is a function of the physiochemical properties of the carrier implant material and of the drug itself. Typically, the implant is made of an inert material which elicits little or no host response.

A controlled release system can be comprised of a neurotoxin incorporated into a carrier. The carrier can be a polymer or a bio-ceramic material. The controlled release system can be injected, inserted or implanted into a selected location of a patient's body and reside therein for a prolonged period during which the neurotoxin is released by the implant in a manner and at a concentration which provides a desired therapeutic efficacy.

Polymeric materials can release neurotoxins due to diffusion, chemical reaction or solvent activation, as well as upon influence by magnetic, ultrasound or temperature change factors. Diffusion can be from a reservoir or matrix. Chemical control can be due to polymer degradation or cleavage of the drug from the polymer. Solvent activation can involve swelling of the polymer or an osmotic effect.

Implants may be prepared by mixing a desired amount of a stabilized neurotoxin into a solution of a suitable polymer dissolved in methylene chloride. The solution may be prepared at room temperature. The solution can then be transferred to a Petri dish and the methylene chloride evaporated in a vacuum desiccator. Depending upon the implant size desired and hence the amount of incorporated neurotoxin, a suitable amount of the dried neurotoxin incorporating implant is compressed at about 8000 p.s.i. for 5 seconds or at 3000 p.s.i. for 17 seconds in a mold to form implant discs encapsulating the neurotoxin.

Preferably, the implant material used is substantially non-toxic, non-carcinogenic, and non-immunogenic. Suitable implant materials include polymers, such as poly(2- hydroxy ethyl methacrylate) (p-HEMA), poly(N-vinyl pyrrolidone) (p-NVP)+, poly(vinyl alcohol) (PVA), poly(acrylic acid) (PM), polydimethyl siloxanes (PDMS), ethylene-vinyl acetate (EVAc) copolymers, polyvinylpyrrolidone/methylacrylate copolymers, polymethylmethacrylate (PMMA), poly(lactic acid) (PLA), poly(glycolic acid) (PGA), polyanhydrides, poly(ortho esters), collagen and cellulosic derivatives and bioceramics, such as hydroxyapatite (HPA), tricalcium phosphate (TCP), and aliminocalcium phosphate (ALCAP). Lactic acid, glycolic acid and collagen can be used to make biodegradable implants.

An implant material can be biodegradable or bioerodible. An advantage of a bioerodible implant is that it does not need to be removed from the patient. A bioerodible implant can be based upon either a membrane or matrix release of the bioactive substance. Biodegradable microspheres prepared from PLA-PGA are known for subcutaneous or intramuscular administration.

A kit for practicing disclosed embodiments is also encompassed by the present disclosure. The kit can comprise a 30 gauge or smaller needle and a corresponding syringe. The kit also comprises a Clostridial neurotoxin composition, such as a botulinum type E toxin composition. The neurotoxin composition may be provided in the syringe. The composition is injectable through the needle. The kits are designed in various forms based the sizes of the syringe and the needles and the volume of the injectable composition contained therein, which in turn are based on the specific deficiencies the kits are designed to treat.

### EXAMPLES

The following non-limiting examples are provided for illustrative purposes only in order to facilitate a more complete understanding of representative embodiments. This example should not be construed to limit any of the embodiments described in the present specification.

### Example 1

### Use of Botulinum Toxin Type E to Treat Glabellar Lines

A randomized, double-blind, placebo- controlled, ascending dose cohort, Phase 2a study in humans was conducted to evaluate safety and efficacy of a single treatment cycle of a disclosed fast-acting type E composition in subjects with glabellar frown lines. This study was conducted in compliance with this protocol and all applicable Federal and State regulations. A composition composed of botulinum neurotoxin subtype E (BoNT/E, "EB-001")

The total dose was divided into 5 injections; 1 injection into procerus at midline and a single injection into each of the medial and lateral corrugators bilaterally, as shown in **FIG. 5****.** The total dose was as follows:

| Cohort | Total BoNT/E Dose (ng) | Dose at Procerus (ng) | Doses at medial corrugators (ng) | Dose at lateral corrugators (ng) |
|---|---|---|---|---|
| 1 | 0.1 | 0.02 | 0.02 into each of right and left corrugators | 0.02 into each of right and left corrugators |
| 2 | 0.3 | 0.06 | 0.06 into each of right and left corrugators | 0.06 into each of right and left corrugators |
| 3 | 0.9 | 0.18 | 0.18 into each of right and left corrugators | 0.18 into each of right and left corrugators |
| 4 | 1.2 | 0.24 | 0.24 into each of right and left corrugators | 0.24 into each of right and left corrugators |
| 5 | 1.6 | 0.32 | 0.32 into each of right and left corrugators | 0.32 into each of right and left corrugators |
| 6 | 2.1 | 0.42 | 0.42 into each of right and left corrugators | 0.42 into each of right and left corrugators |
| 7 | 2.8 | 0.56 | 0.56 into each of right and left corrugators | 0.56 into each of right and left corrugators |

Each subject received 5 injections and the volume injected per site was 0.1 mL. The spacing of injections into the lateral corrugators was at or about 1 centimeter above the supraorbital ridge, which is known to minimize potential ptosis.

### Example 2

### Use of Botulinum Toxin Type E for Brow Lift

A 60 year-old woman presents with eyebrows extending below her brow bone. The physician recommends a composition as disclosed herein, which is injected subdermally above each eye. Each injection site receives about 10 units of type E botulinum toxin, with several injections made on either side of the eye. The drooping of the brow is reduced within about 2 days, and is substantially alleviated for 3 months after administration.

### Example 3

### Use of Botulinum Toxin Type E for Breast Augmentation

A 30 year-old woman elects breast augmentation surgery. 4 hours prior to the procedure, botulinum toxin type E is administered in the proximity of where the surgical incisions will be made. The administration reduces muscle tension in the area of the incision, resulting in minimal scarring. Two weeks after the procedure, a supplemental dose is administered.

### Example 4

### Use of Botulinum Toxin Type E for Reducing Scarring Following Mohs Surgery

A 44 year-old woman is diagnosed with skin cancer, specifically basal cell carcinomas (BCC's) in the forehead area. Other than the BCC's, the patient is in good health, and not pregnant, planning to become pregnant, or lactating. The patient also has no pre-existing disorders of the neuromuscular junction (myasthenia gravis, Eaton-Lambert syndrome, or Amyotrophic Lateral Sclerosis). The patient has not undergone any cosmetic procedure, laser resurfacing treatment, or retinoid therapy in the forehead area in the past 30 days before the Mohs procedure. The patient has no eyebrow or eyelid ptosis at baseline, nor any history of hypertrophic scars or keloid formation or other wound abnormalities as assessed by her doctor.

The patient does not and never has used tobacco, and weighs 140 pounds.

The patient elects to undergo Mohs surgery. The goal of Mohs surgery is to remove as much of the skin cancer as possible, while doing minimal damage to surrounding healthy tissue. Mohs surgery is usually done on an outpatient basis using a local anesthetic.

The roots of a skin cancer may extend beyond the visible portion of the tumor. If these roots are not removed, the cancer will recur. A Mohs procedure starts with the American College of Mohs Surgery (ACMS) specialist examining the visible lesion and planning what tissue to remove. The patient then receives local anesthesia, and the procedure begins.

The procedure is done in stages, all in one visit, while the patient waits between each stage. After removing a layer of tissue, the surgeon examines it under a microscope in an on-site lab. With the help of technicians, the surgeon then color-codes each of these sections with dyes and makes reference marks on the skin to show the source of the sections. A map of the surgical site is then drawn to track exactly where each small portion of tissue originated. If any cancer cells remain, the surgeon removes another layer of tissue from that precise location, while sparing as much healthy tissue as possible. In a laboratory, the surgeon uses a microscope to examine the undersurface and edges of each section of tissue in search of evidence of remaining cancer. If the surgeon finds cancer cells under the microscope, he or she marks their location on the "map" and returns to the patient to remove another deeper layer of skin- but only from precisely where the cancer cells originated. This method ensures that the Mohs surgery results in the smallest scar possible.

The doctor repeats this process until no cancer cells remain.

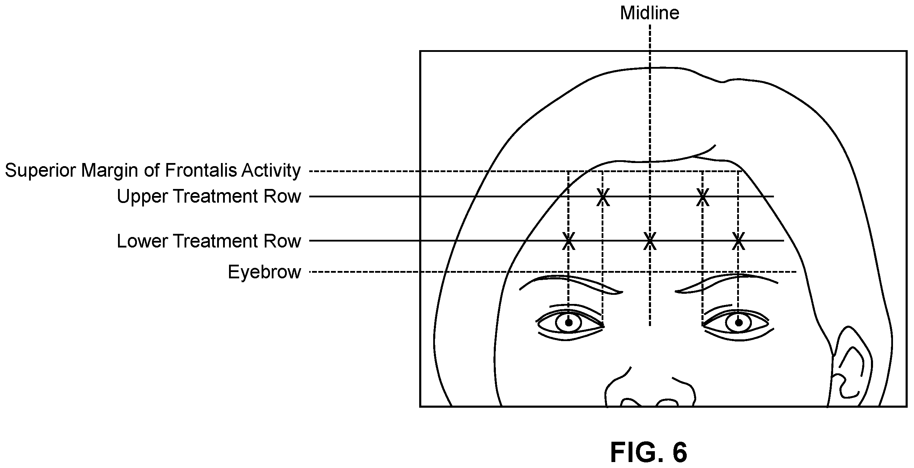

After the surgery, a single 2 cm scar on the patient's forehead is treated with botulinum toxin type E supplied as a sterile solution for injection with a 5-mL vial nominal concentration of 19.1 ng/mL BoNT/E in 0.03 M sodium phosphate, 0.12 M sodium chloride, and 1 mg/mL Human Serum Albumin (HSA), pH 6.0. The total dose of BoNT/E is 2.8 ng, divided over 5 injection sites over two rows (an inverted W pattern; see **FIG. 6****)** into the frontalis muscles. Injection depth for injections to the frontalis is to the subdermal level in order to impact the superficial frontalis muscle tissue, while minimizing the potential for bruising. The needle is superiorly and laterally oriented during the injection, given with needle tip bevel up at a 30-degree angle.

Follow up visits are scheduled at Days 2, 8, 30, and 90. During pre-surgery (prior to dosing) on Day 1, post-surgery on Day 1, and Days 2, 8, 30 (ET), and 90 (EOS), standardized facial photographs will be taken per instructions in the study manual(s).

The follow up visits include:
a. Scar assessment using Visual Analog Scale (VAS)
b. Scar Cosmesis Assessment and Rating Scale (SCARS)
c. Patient and Observer Scar Assessment Scale (POSAS)
d. Investigator assessment of forehead lines, at maximum effort and at rest, using a Facial wrinkle scale (FWS-IA)

The VAS evaluates scar appearance from worst to best using a straight horizontal line of fixed length (10 cm). The ends are defined as the extreme limits of the scar appearance orientated from the left (worst) to the right (best). Raters will make a mark on the line to indicate the appearance of the scar.

The SCARS measure postoperative scare cosmesis with total patient and observer scores ranging from 0 (best possible scar) to 15 (worst possible scar) (Kantor, Reliability and Photographic Equivalency of the Scar Cosmesis Assessment and Rating (SCAR) Scale, an Outcome Measure for Postoperative Scars, JAMA Dermatol. 153(1):55-60, 2017). Scoring of the SCAR scale is based on 6 observer-scored items and 2 patient scored items.

The POSAS includes subjective symptoms of pain and pruritus and expands on the objective data captured in the Vancouver Scar Scale (VSS). It consists of 2 numerical numeric scales: The Patient Scar Assessment Scale and the Observer Scar Assessment Scale. It assesses vascularity, pigmentation, thickness, relief, pliability, and surface area, and it incorporates patient assessments of pain, itching, color, stiffness, thickness, and relief (Draaijers et al., The patient and observer scar assessment scale: a reliable and feasible tool for scar evaluation, Plast Reconstr. Surg. 113(7): 1960-1965, 2004). The POSAS is the only scale that considers subjective symptoms of pain and pruritus, but like other scales it also lacks functional measurements as to whether the pain or pruritus interferes with quality of life. Linear regression analysis has demonstrated that the observer's opinion is influenced by vascularization, thickness, pigmentation, and relief, whereas the patient's opinion is primarily influenced by pruritus and scar thickness. The POSAS has been applied to postsurgical scars and used in the evaluation of linear scars following breast cancer surgery, demonstrating internal consistency and interobserver reliability when compared to the VSS with the added benefit of capturing the patients' ratings.

The FWS-IA is a four-point scale that indicates severity of forehead lines as follows: 0 = none, 1 = mild, 2 = moderate, or 3 = severe. Based on the Investigator assessment, the FWS-IA will be assessed at maximum elevation and at rest.

No Spread of Toxin (SOT) effects are seen at the follow-up visits.

### Example 5

### Use of Botulinum Toxin Type E to Minimize Scarring and to Improve Scar Appearance

Twelve human subjects between the ages of 58-75 years and scheduled to Mohs surgery to remove a single lesion of basal cell carcinoma on the forehead were randomized into a treatment group (n=8) and a placebo group (n=4). The subjects in both groups received a single intramuscular injection in the frontalis muscle near the surgical site, the treatment group receiving a dose of BoNT/E and the placebo group receiving saline. The subjects and the study staff were blinded; the sponsor was unblinded. The table below summarizes the demographics of the subjects.

| **Treatment Group** | **Subject ID** | **Gender** | **Age** | **Mean/Median Age** | **Gender Distribution (M/F)** | **Surgical Stages** |
|---|---|---|---|---|---|---|
| Placebo | 101-009 | Female | 67 | 67/66 | 50/50 | 2 |
| | 101-010 | Male | 75 | | | 2 |
| | 101-004 | Female | 60 | | | 3 |
| | 101-001 | Male | 64 | | | 1 |
| BoNT/E | 101-002* | Female | 66 | 59/64 | 62/38 | 2 |
| | 101-013 | Male | 63 | | | 2 |
| | 101-005 | Male | 64 | | | 2 |
| | 101-003 | Male | 47 | | | 2 |
| | 101-006 | Female | 34 | | | 2 |
| | 101-011 | Male | 72 | | | 2 |
| | 101-012 | Female | 58 | | | 3 |
| | 101-007 | Male | 71 | | | 4 |

| | | | | | | |
|---|---|---|---|---|---|---|
| *Subject removed from analysis due to high tension closure to wound dehiscence. | | | | | | |

On Day 1 of the study, the subjects were admitted for the surgical procedure and the placebo or the BoNT/E was administered via injection intramuscularly. Surgery was conducted to remove the carcinoma and the wound (about 2 cm in length) was closed. On Day 2 of the study, 24 hours after surgery, each subject was assessed using the VAS, SCAR and POSAS scales (see Example 4). On Day 8, the subjects were again assessed using the various scales and the external sutures were removed. On Day 30 and on Day 90 the subjects were again assessed using the various scales, and the study formally concluded on Day. 90. Results are shown in **FIGS. 7A-7C****.**

In closing, it is to be understood that although aspects of the present specification are highlighted by referring to specific embodiments, one skilled in the art will readily appreciate that these disclosed embodiments are only illustrative of the principles of the subject matter disclosed herein. Therefore, it should be understood that the disclosed subject matter is in no way limited to a particular methodology, protocol, and/or reagent, etc., described herein. As such, various modifications or changes to or alternative configurations of the disclosed subject matter can be made in accordance with the teachings herein without departing from the spirit of the present specification. Lastly, the terminology used herein is for the purpose of describing particular embodiments only, and is not intended to limit the scope of the present disclosure, which is defined solely by the claims. Accordingly, embodiments of the present disclosure are not limited to those precisely as shown and described.

Certain embodiments are described herein, comprising the best mode known to the inventor for carrying out the methods and devices described herein. Of course, variations on these described embodiments will become apparent to those of ordinary skill in the art upon reading the foregoing description. Accordingly, this disclosure comprises all modifications and equivalents of the subject matter recited in the claims appended hereto as permitted by applicable law. Moreover, any combination of the above-described embodiments in all possible variations thereof is encompassed by the disclosure unless otherwise indicated herein or otherwise clearly contradicted by context. Specific embodiments disclosed herein may be further limited in the claims using consisting of or consisting essentially of language. When used in the claims, whether as filed or added per amendment, the transition term "consisting of" excludes any element, step, or ingredient not specified in the claims. The transition term "consisting essentially of" limits the scope of a claim to the specified materials or steps and those that do not materially affect the basic and novel characteristic(s). Embodiments of the present disclosure so claimed are inherently or expressly described and enabled herein.

### Embodiments

1. A method for minimizing scarring, comprising:
   administering a therapeutically effective amount of a fast-acting neurotoxin to a patient in the proximity of a wound.
2. The method of item 1, wherein said fast-acting neurotoxin comprises botulinum neurotoxin serotype E.
3. The method of item 1 or item 2, wherein said therapeutically effective amount comprises an amount of between about 10⁻³ U/kg and about 35 U/kg.
4. The method of item 1 or item 2, wherein said therapeutically effective amount comprises an amount of between about 0.2 nanograms and about 2 nanograms.
5. The method of any preceding item , wherein said administering comprises administering by injection.
6. The method of item 5, wherein said administering by injection is an intramuscular injection.
7. The method of any preceding item , wherein said method further comprises administering a botulinum toxin subtype A to the patient.
8. The method of any one of items 1-6, wherein said method further comprises administration of a slower-acting neurotoxin to the patient.
9. The method of any one of items 1-6, wherein said method further comprises administration of a slower-recovery neurotoxin to the patient.
10. The method of any preceding item , wherein said wound comprises a surgical incision.
11. The method of item 10, wherein said surgical incision is a result of a cosmetic surgical procedure.
12. The method of items 10, wherein said surgical incision is a result of a Mohs procedure.
13. A method for improving appearance of a scar that forms over a wound, comprising:
   administering a therapeutically effective amount of botulinum toxin subtype E (BoNT/E) in the proximity of a wound.
14. The method of item 13, wherein the wound is a surgical wound.
15. The method of item 13 or item 14, wherein said administering is performed before a surgical procedure.
16. The method of any one of items 13-15, wherein said administering is performed after a surgical procedure.
17. The method of item 14, wherein said surgical wound overlaps with an existing scar on the subject.
18. The method of any one of items 13-17, wherein said therapeutically effective amount comprises an amount that reduces redness of the scar, relative to a scar that forms over a similar wound in a patient not treated with BoNT/E.
19. The method of any one of items 13-18, wherein said therapeutically effective amount comprises an amount that reduces stiffness of the scar, relative to a scar that forms over a similar wound in a patient not treated with BoNT/E.
20. The method of any one of items 13-19, further comprising administering botulinum toxin subtype A in the proximity of the wound.

## Claims

1. A fast-acting neurotoxin for use in a method of minimizing scarring, the method comprising administering a therapeutically effective amount of the fast-acting neurotoxin to a patient in the proximity of a wound.

2. The fast-acting neurotoxin for use of claim 1, wherein said fast-acting neurotoxin comprises botulinum neurotoxin serotype E.

3. The fast-acting neurotoxin for use of claim 1 or claim 2, wherein said therapeutically effective amount is between about 10⁻³ U/kg and about 35 U/kg.

4. The fast-acting neurotoxin for use of claim 1 or claim 2, wherein said therapeutically effective amount is between about 0.2 nanograms and about 2 nanograms.

5. The fast-acting neurotoxin for use of any preceding claim, wherein said administering comprises administering by injection.

6. The fast-acting neurotoxin for use of claim 5, wherein said injection is an intramuscular injection.

7. The fast-acting neurotoxin for use of any preceding claim, wherein said method further comprises administering a botulinum toxin subtype A to the patient.

8. The fast-acting neurotoxin for use of any of claims 1-6, wherein said method further comprises administration of a slower-acting neurotoxin to the patient.

9. The neurotoxin for use of any of claims 1-6, wherein said method further comprises administration of a slower-recovery neurotoxin to the patient.

10. The fast-acting neurotoxin for use of any preceding claim, wherein said wound comprises a surgical incision.
